# EUROPÄISCHE PATENTSCHRIFT

(11) **EP 0 581 788 B1**
(45) Veröffentlichungstag und Bekanntmachung des Hinweises auf die Patenterteilung: **02.10.1996**
(21) Anmeldenummer: 92907667.7
(22) Anmeldetag: 13.04.1992
(51) Int. Cl.: A61M 5/315

(54) **INJEKTIONSGERÄT**
INJECTION DEVICE
DISPOSITIF D'INJECTION

(30) Priorität: 15.04.1991 DE 4112259
(43) Veröffentlichungstag der Anmeldung: 09.02.1994
(73) Patentinhaber: MEDICO DEVELOPMENT INVESTMENT COMPANY, CH-6612 Ascona (CH)
(72) Erfinder: GABRIEL, Jochen, D-7000 Stuttgart 1 (DE)
(74) Vertreter: Raible, Hans, Dipl.-Ing.
(86) Internationale Anmeldenummer: EP9200831
(87) Internationale Veröffentlichungsnummer: WO9218179

(56) Entgegenhaltungen:
- EP-A- 0 058 536
- EP-A- 0 265 876
- EP-A- 0 268 191
- WO-A-88/08725

## Beschreibung

Die Erfindung betrifft ein Injektionsgerät mit einem zur Aufnahme von Injektionsflüssigkeit dienenden Gehäuse, mit einem längenverstellbaren Stößel, der beim Injektionsvorgang zwischen einer ersten Stellung, im folgenden Dosierstellung genannt, und einer zweiten Stellung, im folgenden Ruhestellung genannt, verschiebbar ist, um Injektionsflüssigkeit auszupressen, und mit einem Stellorgan, welches in der Dosierstellung mittels einer dort aktivierten Wirkverbindung eine Veränderung der wirksamen Länge dieses Stößels und damit ein Dosieren der injizierten Menge von Injektionsflüssigkeit ermöglicht.

Ein derartiges Injektionsgerät ist bekannt aus der DE-C 3 638 984 (EP-A-0 268 191). Hierbei handelt es sich um einen vollautomatischen Injektor mit Federsystem. Bei diesem Gerät ist es möglich, die wirksame Länge des Stößels in allen Betriebsstellungen zu verändern. Wenn also jemand mit dem Gerät herumspielt - was nie zu vermeiden ist -, besteht das Risiko, daß durch Herumdrehen die Länge des Stößels verändert wird, was zu Fehldosierungen führen kann.

Ein anderes Injektionsgerät der eingangs genannten Art ist bekannt aus der WO 88/08725. Auch hier handelt es sich um einen vollautomatischen Injektor mit Federsystem, und auch bei diesem bekannten Gerät ist es möglich, die wirksame Länge des Stößels in allen Betriebsstellungen zu ändern, also auch in der Ruhestellung. Dies ist bei diesem Gerät deshalb erforderlich, damit in der Ruhestellung zur Kontrolle etwas Injektionsflüssigkeit ausgepreßt werden kann. Beim Herumspielen, z.B. durch Kinder, kann aber unter Umständen versehentlich viel Insulin ausgepreßt werden. Dieses bekannte Gerät hat den Vorteil, daß - durch verschiedene Sicherheitsvorkehrungen - die eigentliche Dosiseinstellung erst dann möglich ist, wenn sich das Gerät in seiner gespannten Stellung befindet. Dies wird freilich erkauft durch eine relativ komplizierte Bedienung.

Aus der US-A-4 865 591 kennt man ebenfalls ein Injektionsgerät mit einem längsverschiebarem Stößel. Zum Einstellen der Dosis dienen hier Klemmbacken. Diese werden nach einer Injektion außer Eingriff mit dem Stößel gebracht, in distaler Richtung verschoben u. dann an der neuen Stellung in Eingriff mit einer Verzahnung des Stößels gebracht. Durch Druck auf die Klemmbacken in proximaler Richtung kann dann injiziert werden. Die Bedienung dieses Geräts ist durch die vielen erforderlichen Schritte sehr kompliziert.
Die Dosiereinstellung ist sowohl in der ersten wie in der zweiten Stellung aktiviert. Man kann sie auch in beiden Stellungen desaktivieren, allerdings durch Drehung eines separaten Betätigungsglieds.

Die Aufgabe der Erfindung wird deshalb darin gesehen, ein neues, einfach zu bedienendes Injektionsgerät bereitzustellen, das unbeabsichtige Fehldosierung nicht zuläßt.

Nach der Erfindung wird diese Aufgabe dadurch gelöst, daß in der Ruhestellung diese Wirkverbindung gesteuert durch die Längen-Verschiebung des längenverstellbaren Stößels von der Dosierstellung in die Ruhestellung desaktiviert ist. nicht aktiviert ist. Man kann also in der Ruhestellung, die das Gerät nach einer Injektion einnimmt, beliebig an ihm herumdrehen, ohne daß sich dadurch die eingestellte Dosis ändert, denn die Wirkverbindung, welche der Stellanordnung eine Veränderung der wirksamen Länge des Stößels ermöglicht, ist in dieser Ruhestellung nicht aktiviert.

Dies geschieht in bevorzugter Weise dadurch, daß in der Ruhestellung die Wirkverbindung unterbrochen ist. Nach einer derzeit weniger bevorzugten Alternative könnte die Veränderung der wirksamen Länge des Stößels in dieser Stellung blockiert sein.

Dabei geht man mit besonderem Vorteil so vor, daß zwischen längenverstellbarem Stößel und Gehäuse eine Sperre vorgesehen ist, welche nur in einer bestimmten Drehstellung oder einem bestimmten Drehstellungsbereich des längenverstellbaren Stößels eine axiale Verschiebung desselben relativ zum Gehäuse von seiner Ruhestellung in seine Dosierstellung ermöglicht. Man kann also das Injektionsgerät nur in einer vorbestimmten Drehstellung des längenverstellbaren Stößels in seine Dosierstellung bringen. Da die Dosiseinstellung erst in dieser Dosierstellung möglich ist, erreicht man, daß die Dosierung immer von der gleichen Nullstellung aus beginnt, die gewöhnlich am Gehäuse mit einem geeigneten Symbol markiert ist. Die Einstellung der Dosis erfolgt dann durch Betätigen des Stellorgans bis zum gewünschten Dosiswert, der auf einer Skala abgelesen oder in der üblichen Weise anhand von Klickgeräuschen gezählt werden kann.

Man bildet dabei das Injektionsgerät bevorzugt so aus, daß die Sperre bei einer Bewegung des längenverstellbaren Stößels von seiner Dosierstellung in seine Ruhestellung nicht wirksam ist. Es handelt sich also in diesem Fal um eine Sperre, die nur in einer Richtung wirksam ist und die den Injektionsvorgang, unabhängig von der eingestellten Dosis, nicht behindert.

Insbesondere bei Verwendung dieser Sperre ergibt sich in sehr vorteilhafter Weise die Möglichkeit, dem Stellorgan einen in der Dosierstellung wirksamen Anschlag zuzuordnen, welcher die einstellbare Dosis nach oben begrenzt. Dieser Anschlag kann verstellbar ausgebildet sein, wodurch es möglich wird, die maximal einstellbare Dosis nach den Bedürfnissen des Patienten individuell einzujustieren. Man kann so in sehr einfacher Weise die maximale Dosis nach oben begrenzen, und der Arzt kann z.B. ein Injektionsgerät mit einer bestimmten Maximaldosis verschreiben, um Fehlern durch den Patienten nach Möglichkeit vorzubeugen. Selbstverständlich sind unterhalb dieser Maximaldosis kleinere Dosiseinstellungen weiterhin möglich.

Eine sehr einfache Ausgestaltung der Erfindung ist dadurch gekennzeichnet, daß der längenverstellbare Stößel eine in einem Gewinde eines Gewindeglieds geführte Gewindespindel aufweist, mit welchem Gewindeglied ein zur Längsführung der Gewindespindel ausgebildetes Führungsglied drehbar verbunden ist, welches im Gehäuse axial verschiebbar angeordnet ist. Die Längenverstellung des Stößels wird hierdurch sehr einfach und mit einem geringen Aufwand an Teilen möglich.

Eine sehr vorteilhafte Ausgestaltung der Erfindung ist dadurch gekennzeichnet, daß zwischen Gewindeglied und Führungsglied eine Ratschenanordnung vorgesehen ist. Diese Ratschenanordnung bewirkt, daß der längenverstellbare Stößel seine vom Benutzer eingestellte Länge bei der Injektion sicher beibehält, denn sie sperrt - außer bei der Dosiseinstellung - eine Verdrehung zwischen Führungsglied und Gewindeglied und blockiert dadurch eine Verschiebung der Gewindespindel aus ihrer eingestellten Lage heraus. Andererseits ermöglicht diese Ratschenanordnung eine Verstellung der wirksamen Länge des Stößels in diskreten Schritten, z.B. in Schritten von einer zu injizierenden Einheit, wenn sich das Gerät in seiner Arbeitsstellung befindet, und sie erzeugt beim Verstellen klickende Geräusche, die vom Anwender gezählt werden können.

Eine sehr vorteilhafte Weiterbildung der Erfindung ist dadurch gekennzeichnet, daß das Führungsglied in der Ruhestellung relativ zum Gehäuse verdrehbar, dagegen in der Dosierstellung relativ zum Gehäuse nicht verdrehbar ist. Solange sich das Führungsglied in der Ruhestellung befindet und dadurch verdrehbar ist, ist eine Änderung der wirksamen Länge des Stößels - und damit eine Dosiseinstellung - nicht möglich. Befindet sich aber das Führungsglied in der Dosierstellung, so ist es relativ zum Gehäuse nicht verdrehbar, und die Dosis kann eingestellt werden. Die drehfeste Verbindung in der Dosierstellung kann in bevorzugter Weise dadurch erreicht werden, daß das Führungsglied in der Dosierstellung form- oder kraftschlüssig mit dem Gehäuse verbunden ist, wobei man insbesondere so vorgeht, daß das Führungsglied mit einer Vertiefung oder einem Vorsprung versehen ist, mit der bzw. dem in der Dosierstellung ein hierzu passender Vorsprung bzw. eine hierzu passende Vertiefung des Gehäuses in Eingriff steht, nicht aber in der Ruhestellung, wodurch in der Ruhestellung die Wirkverbindung zur Veränderung der wirksamen Lage des Stößels unterbrochen wird.

Eine sehr vorteilhafte Weiterbildung der Erfindung ist ferner dadurch gekennzeichnet, daß die Drehung zwischen Gewindeglied und Führungsglied auf eine Drehrichtung beschränkt ist, welche eine Verlängerung der wirksamen Länge des Stößels bewirkt. Dies verhindert, daß aus Versehen die Dosierung in die falsche Richtung erfolgt, in der die wirksame Länge des Stößels verkürzt würde und der Patient folglich kein Medikament erhalten würde. Der Patient kann also nur in der "richtigen" Richtung dosieren - in der falschen Richtung "sperrt" das Gerät. Dabei kann man mit Vorteil so vorgehen, daß die Ratschenanordnung gegen eine Drehung in der unerwünschten Drehrichtung sperrend ausgebildet ist. Die Ratschenanordnung ermöglicht in diesem Fall nur eine Verdrehung in einer einzigen Richtung, welche eine Vergrößerung der wirksamen Länge des Stößels bewirkt.

Weitere Einzelheiten und vorteilhafte Weiterbildungen der Erfindung ergeben sich aus dem im folgenden beschriebenen und in der Zeichnung dargestellten, in keiner Weise als Einschränkung der Erfindung zu verstehenden Ausführungsbeispiel. Es zeigt:
- Fig. 1: eine Seitenansicht eines erfindungsgemäßen Injektionsgeräts,
- Fig. 2: einen Längsschnitt durch das Injektionsgerät der Fig. 1 in seiner Ruhestellung, also nach Abschluß einer Injektion,
- Fig. 3: einen Längsschnitt durch das Injektionsgerät der Figuren 1 und 2 in seiner Dosierstellung, im folgenden auch Arbeitsstellung genannt, aber vor dem Einstellen einer zu injizierenden Dosis,
- Fig. 4: einen Längsschnitt analog Fig. 3, aber nach dem Einstellen der zu injizierenden Dosis,
- Fig. 5: einen Längsschnitt durch das Gerät der Fig. 4, aber nach Abschluß des Injektionsvorganges,
- Fig. 6: eine raumbildliche Darstellung der für die Dosiseinstellung verwendeten Teile des Geräts, in Einzeldarstellung,
- Fig. 6A: eine Variante zu Fig. 6c),
- Fig. 7: eine raumbildliche Darstellung der für die Dosiseinstellung verwendeten Teile des Geräts im zusammengebauten Zustand und in der Stellung analog Fig. 2,
- Fig. 8: eine raumbildliche Darstellung analog Fig. 7, aber in der Stellung analog Fig. 4, also beim Einstellen der zu injizierenden Dosis,
- Fig. 9: einen Längsschnitt durch den für die Dosiseinstellung verwendeten Teil des Geräts, in vergrößertem Maßstab, und
- Fig. 10 - 14: Schnitte, gesehen längs der Linien X-X bis XIV-XIV der Fig. 9.

In der nachfolgenden Beschreibung beziehen sich die Begriffe links, rechts, unten, oben etc. auf die jeweilige Figur. Die Begriffe proximal und distal werden wie in der Medizin üblich gebraucht, also proximal = dem Patienten zugewandt und distal = vom Patienten abgewandt.

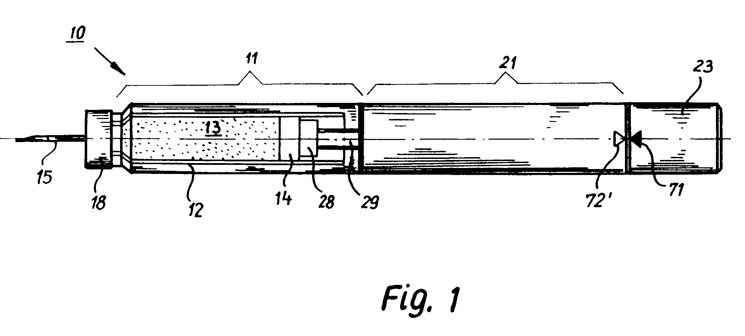

Fig. 1 zeigt ein erfindungsgemäßes Injektionsgerät 10 in der Seitenansicht und etwa im Maßstab 1 : 1. Sein linker Gehäuseteil 11 ist aus durchsichtigem Material ausgebildet und enthält in seinem Inneren eine Kartusche 12, die gewöhnlich aus Glas besteht und mit der zu injizierenden Flüssigkeit 13 gefüllt ist, z.B. mit Papaverin oder Insulin. Rechts, bezogen auf Fig. 1, wird diese Flüssigkeit begrenzt durch einen in axialer Richtung verschiebbaren Kolben 14 aus einem geeigneten elastischen Material, gewöhlich Gummi. Wird dieser Kolben 14 nach links verschoben, so wird Flüssigkeit aus der Kartusche 12 gepreßt und tritt durch eine Injektionsnadel 15, auch Kanüle genannt, aus. Der Weg, um den der Kolben 14 bei einer Injektion nach links verschoben wird, bestimmt die Quantität der injizierten Flüssigkeit. Der Benutzer kann die Lage des Kolbens 14 visuell durch das Gehäuseteil 11 hindurch beobachten und weiß daher, wieviel Injektionsflüssigkeit noch zur Verfügung steht.

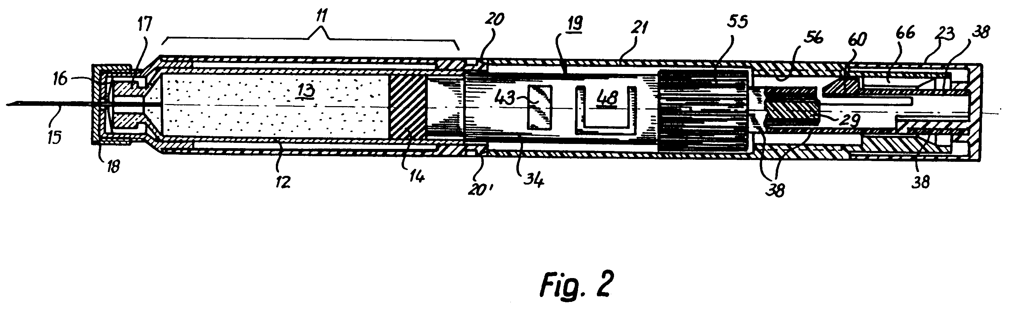

Die Kartusche 12 verjüngt sich, wie in Fig. 2 dargestellt, an ihrem linken Ende und ist dort mit einer Metallkappe 17 versehen, die eine dünne Gummimembran 16 festhält. Die Injektionsnadel 15 ist in einer Gewindekappe 18 gehaltert, und wenn diese auf ein Außengewinde am proximalen Ende des Gehäuseteils 11 aufgeschraubt ist, durchsticht das distale Ende der Nadel 15 die Gummimembran 16 und dringt in die Injektionsflüssigkeit 13 ein. Die Nadel 15 kann so - durch Abschrauben der Gewindekappe 18 - leicht ausgetauscht werden, um für jede Injektion eine sterile Nadel zur Verfügung zu haben.

Der linke Gehäuseteil 11 ist, wie dargestellt, durch Rastverbindungen 20 mit einem rechten Gehäuseteil 21 verbunden, der - wie der linke Gehäuseteil 11 - aus einem geeigneten Kunststoff ausgebildet sein kann, der aber hier nicht transparent zu sein braucht. Im Gehäuseteil 21 befindet sich die Mechanik 19 für die Einstellung der zu injizierenden Flüssigkeitsmenge und für die Durchführung des eigentlichen Injektionsvorgangs, also des Vorgangs, der auf das Einstechen der Nadel 15 in das Gewebe des Patienten folgt und bei dem die Flüssigkeit in dieses Gewebe injiziert wird.

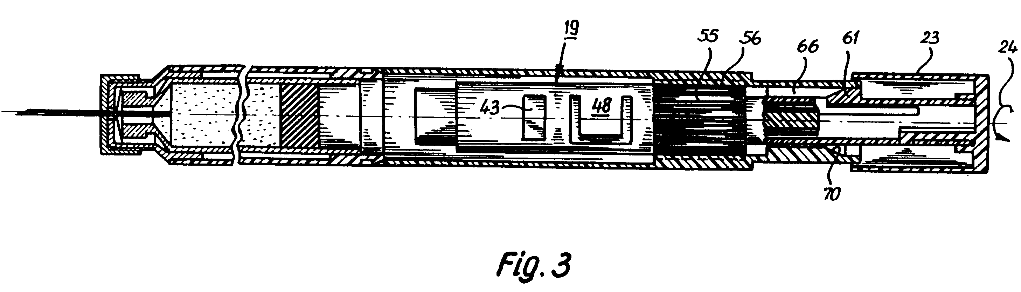

Zur Einstellung der zu injizierenden Flüssigkeitsmenge und zum Durchführen des Einspritzvorgangs dient ein Stellglied 23 in Form eines Betätigungsknopfes. Wie ein Vergleich von Fig. 2 mit Fig. 3 zeigt, kann dieses Stellglied 23 in axialer Richtung verschoben werden. Es kann ferner in der Dosierstellung, im folgenden auch Arbeitsstellung genannt, zur Einstellung der zu injizierenden Flüssigkeitsmenge verdreht werden, wie in Fig. 3 durch einen Pfeil 24 angedeutet.

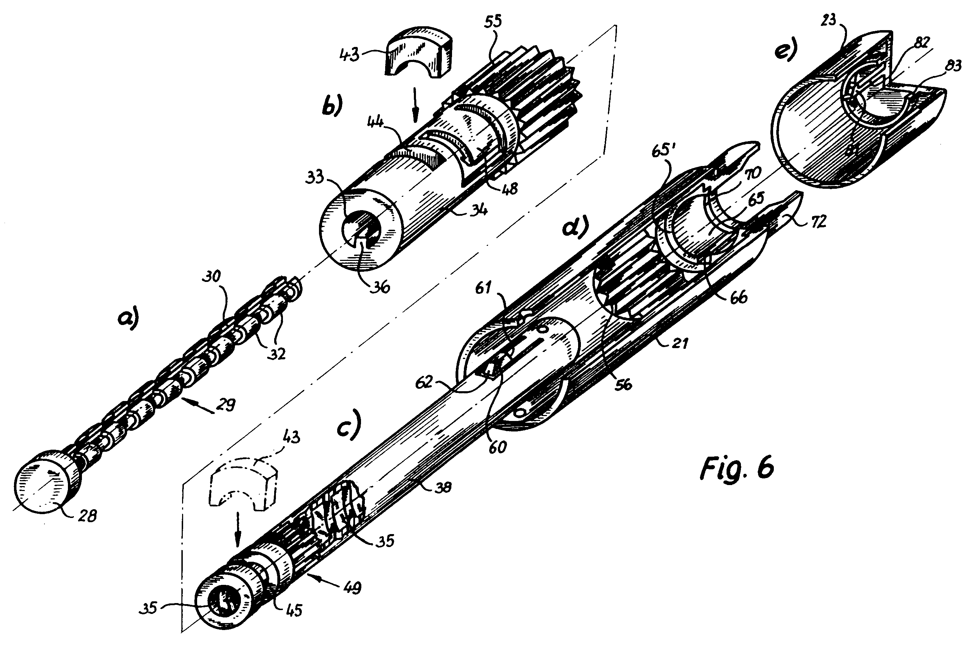

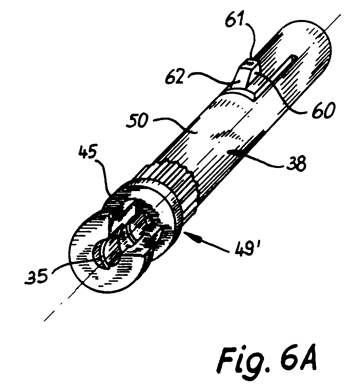

Die Fig. 6 bis 8 zeigen raumbildlich, und dadurch leichter verständlich, die Mechanik 19 und ihre Teile. Die Mechanik 19 besteht nur aus wenigen Teilen, die gewöhnlich als Spritzgußteile billig aus einem geeigneten Kunststoff hergestellt werden.

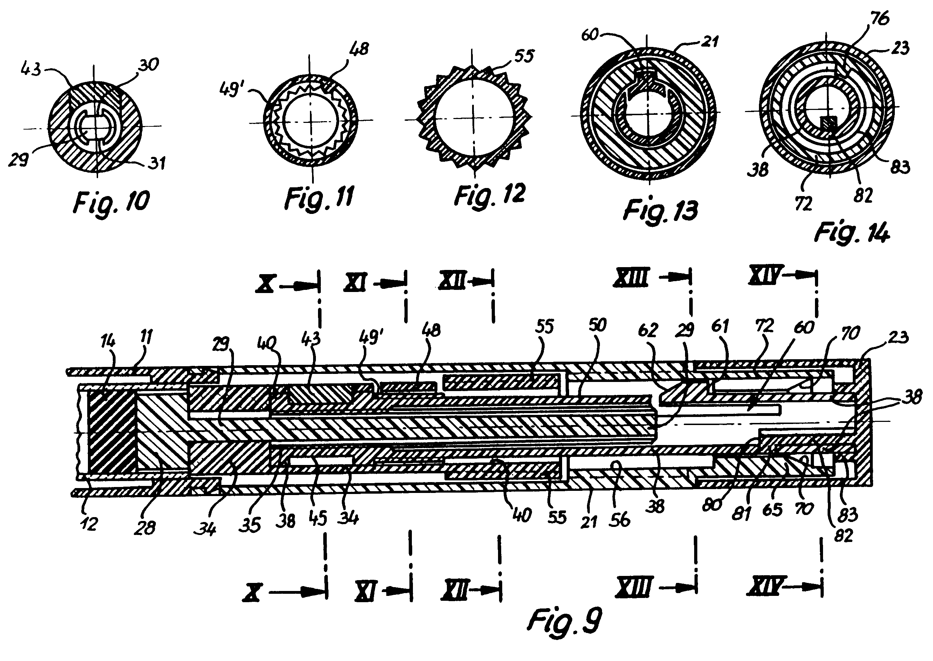

Direkt auf den Kolben 14 wirkt das proximale Ende 28 einer Gewindespindel 29 (Fig. 6a), die bevorzugt zwei gegenüberliegende Längsnuten 30, 31 (Fig. 10) aufweist und die auf ihrer Außenseite mit einem Rechteck-Steilgewinde 32 versehen ist. Die Gewindespindel 29 ist aus Kunststoff gespritzt, und die beiden gegenüberliegenden Längsnuten 30, 31 verhindern, daß sie sich nach dem Herausnehmen aus der Spritzform verzieht, also krumm wird, was ihre Funktion gefährden könnte.

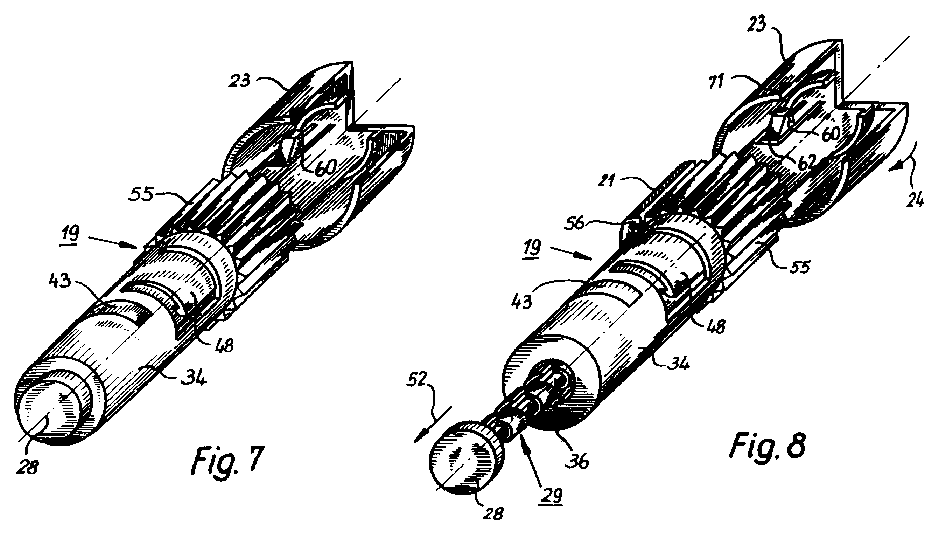

Die Gewindespindel 29 wird durchgesteckt durch eine axiale Öffnung 33 eines Führungsgliedes 34, und sie wird in dieser axialen Öffnung 33 in Längsrichtung geführt durch eine dort vorgesehene Führungsnase 36 (Fig. 8), welche in einer der Längsnuten 30 bzw. 31 der Gewindespindel 29 läuft.

Nach dem Durchstecken durch die axiale Öffnung 33 (Fig. 6) wird die Gewindespindel 29 eingeschraubt in das Innengewinde 35 eines Gewindeglieds 38. Letzteres wird dabei in eine axiale Ausnehmung 40 (Fig. 9) des Führungsgliedes 34 hineingezogen und wird, wenn es dort seine Endlage erreicht hat, durch ein Arretierglied 43 gegen axiale Verschiebung relativ zum Führungsglied 34 gesichert. Das Arretierglied 43 wird von außen durch eine Öffung 44 (Fig. 6b) des Führungsgliedes 34 eingeführt und greift mit seinem inneren Ende in eine Ringnut 45 am proximalen Ende des Gewindeglieds 38 ein. Gewindeglied 38 und Führungsglied 34 können sich also relativ zueinander verdrehen, aber nicht relativ zueinander verschieben, mit Ausnahme des unvermeidlichen Spiels bei solchen Anordnungen.

Wenn also zur Dosierung das Gewindeglied 38 in der Arbeitsstellung verdreht wird, wie in Fig. 3 durch einen Pfeil 24 angedeutet, dann würde sich an sich auch die Gewindespindel 29 mitdrehen. Wenn jedoch das Führungsglied 34 in der Arbeitsstellung im Gehäuseteil 21 nicht drehbar arretiert ist, kann sich die Gewindespindel 29 relativ zum Gehäuseteil 21 nicht drehen, sondern ist hierbei zu einer axialen Bewegung gezwungen.

Die Verdrehung zwischen dem Gewindeglied 38 und dem Führungsglied 34 ist ebenfalls begrenzt, und zwar durch eine Ratschenanordnung mit einer Rastklinke 48 am Führungsglied 34, welche Rastklinke in eine Rastverzahnung 49 am Gewindeglied 38 eingreift. Bei der Montage wird diese Rastanordnung 48, 49 durch Anheben der Rastklinke 48 gelöst. Sie ermöglicht anschließend - bevorzugt - nur noch eine Drehung des Stellglieds 23 entgegen dem Uhrzeigersinn, wenn man von der distalen Seite auf dasselbe schaut, sperrt aber gegen eine Verdrehung im Uhrzeigersinn. Folglich kann die Gewindespindel 29 nur aus dem Gewindeglied 38, mit dem sie zusammen einen längenverstellbaren Stößel 29, 38 bildet, herausgedreht werden, jedoch nicht in das Gewindeglied 38 hineingedreht werden. Diese Längenverstellung des Stößels 29, 38 ist aber nur möglich, wenn dabei das Führungsglied 34 im Gehäuseteil 21 gegen Verdrehung gesichert ist.

Fig. 6A zeigt eine Variante des in Fig. 6c) dargestellten Gewindeglieds 38. Das Innengewinde 35 ist hier deutlicher dargestellt. Die Rastverzahnung 49' ist hier gleich ausgebildet wie in den Fig. 9 und 11. Sie ermöglicht eine Dosiseinstellung in beiden Richtungen, d.h. wenn der Patient die Dosis seines Medikaments zu hoch eingestellt hat, kann er die Dosiseinstellung wieder etwas zurückdrehen, was bei der Ausführungsform nach Fig. 6c) nicht möglich ist. Der zylindrische Abschnitt 50, der sich in distaler Richtung an die Verzahnung 49' anschließt, hat bevorzugt einen kleineren Durchmesser als diese, wie in den Fig. 6A und 9 dargestellt. Dies erleichtert die Herstellung des Gewindeglieds 38 als Spritzgußteil. Die Teile 45 und 60, 61, 62 der Fig. 6A stimmen mit Fig. 6c) überein und werden deshalb nicht nochmals beschrieben.

Nach der Erfindung ist die Sicherung des Führungsglieds 34 gegen Verdrehung relativ zum Gehäuseteil 21 nur in einem Teil der möglichen axialen Stellungen der Mechanik 19 gegeben, d.h. die Längenverstellung ist nicht überall möglich.

Zu diesem Zweck ist beim Ausführungsbeispiel auf der Außenseite des Führungsglieds 34, und an dessen distalem Ende, eine Außenverzahnung 55 vorgesehen, und dieser ist im Inneren des Gehäuseteils 21 eine korrespondierende Innenverzahnung 56 zugeordnet.

Wie Fig. 9 klar zeigt, steht in der Stellung nach einer Injektion, die auch als Ruhestellung bezeichnet werden kann, die Außenverzahnung 55 (auf dem Führungsglied 34) nicht in Eingriff mit der Innenverzahnung 56 (im Inneren des Gehäuseteils 21), d.h. in dieser Stellung, die auch in Fig. 2 dargestellt ist, bewirkt eine Drehung des Stellglieds 23 nur eine Drehung der Mechanik 19 einschließlich der Gewindespindel und ihres proximalen Endes 28, aber nicht das Herausschrauben der Gewindespindel 29 aus dem Gewindeglied 38. Dies ist deshalb wichtig, weil die Menschen vielfach

unbewußt an Gegenständen herumspielen und daher auch die Tendenz bestehen wird, an diesem Gerät und seinen Teilen herumzudrehen, und weil das in dieser Ruhestellung ohne Gefahr möglich ist und das Gerät dadurch nicht verstellt werden kann. Eine Verstellung der Gesamtlänge des Stößels 29, 38 setzt nämlich notwendig eine Verdrehung des Gewindeglieds 38 relativ zum Führungsglied 34 voraus, und diese ist nur möglich, wenn das Führungsglied 34 durch das Gehäuseteil 21 festgehalten wird. Dies aber ist in der Ruhestellung nicht der Fall, d.h. in der Ruhestellung ist eine Einstellung der Injektionsdosis nicht möglich.

An seinem distalen Endbereich ist das Gewindeglied 38 in der dargestellten Weise mit einem federnd auslenkbaren Sperrglied 60 versehen, das auf seiner distalen Seite 61 als Sperre und auf seiner proximalen Seite 62 mit einer Schräge ausgebildet ist, vgl. insbesondere Fig. 9.

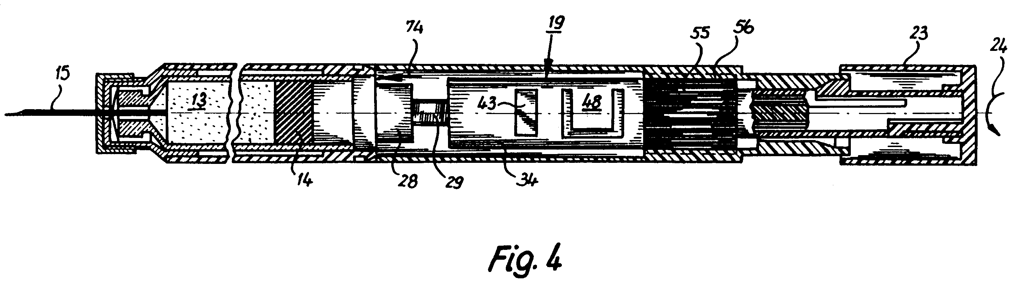

Dem Sperrglied 60 ist ein Sperrgürtel 65 im Inneren des distalen Endes des Gehäuseteils 21 zugeordnet. Dieser Sperrgürtel 65 hat die Form eines radial nach innen vorstehenden Ringwulstes, der nur an einer Stelle einen Durchlaß in Form einer Längsnut 66 hat, deren Querschnittsform an diejenige des Sperrglieds 65 angepaßt ist. Nur an der Stelle dieser Längsnut 66 kann also das Sperrglied 60 den Sperrgürtel 65 überwinden, wenn das Gewindeglied 38 von seiner Ruhestellung (Fig. 2) in seine Arbeitsstellung (Fig. 3 und 4) verschoben werden soll.

Hierdurch wird also erzwungen, daß die Verschiebung der Mechanik 19 von der Ruhestellung in die Arbeitsstellung nur in einer bestimmten Drehstellung dieser Mechanik 19 relativ zum Gehäuseteil 21 möglich ist.

Die Längsnut 66 hat aber auch eine weitere Funktion, denn sie führt das Stellglied 23 so lange, bis dieses seine Arbeitsstellung erreicht hat und ermöglicht erst dann eine Einstellung der Dosis. Solange sich nämlich das Sperrglied 60 in der Längsnut 66 befindet, verhindert diese jede Drehung des Stellglieds 23. Dies zwingt den Patienten, das Stellglied 23 voll in die Arbeitsstellung zu verschieben, da erst dort eine Dosiseinstellung möglich wird.

Wie nämlich Fig. 3 besonders gut zeigt, tritt das Sperrglied 60 bei Erreichen der Arbeitsstellung aus der Längsnut 66 aus und gelangt in eine kegelstumpfförmige Ausnehmung 70 am distalen Ende des Sperrgürtels 65, deren Kegelwinkel an die Neigung der Schräge 62 angepaßt ist, so daß sich diese in der Ausnehmung 70 drehen kann und man dort, also nach Erreichen der Arbeitsstellung, die Dosis durch Verdrehen des Stellglieds 23 einstellen kann.

In dieser Strellung, also dann, wenn das Sperrglied 60 die Längsnut 66 verlassen hat, hat das Gerät seine Nullstellung, und ein am Stellglied 23 angebrachtes Symbol 71 (Fig. 1) kann z.B. in dieser Stellung der Nullstellung einer (nicht dargestellten) Skala gegenüberstehen, die auf der Außenseite des distalen Endbereichs 72 des Gehäuseteils 21 aufgebracht ist. In der Ruhestellung wird dieser distale Endbereich 72 vom Stellglied 23 überdeckt und ist dann nicht sichtbar. Gemäß Fig. 1 kann am Gehäuseteil 21 zusätzlich eine Markierung 72' vorgesehen sein.

In der Arbeitsstellung, wie sie in den Figuren 3 und 4 dargestellt ist, kann gemäß Fig. 4 durch Verdrehen des Stellglieds 23 in Richtung des Drehpfeiles 24 das proximale Ende 28 der Gewindespindel 29 in Richtung des Pfeiles 74 verstellt werden, also in proximaler Richtung. Dieses Verdrehen des Stellgliedes 23 in Richtung des Drehpfeiles 24 ist bevorzugt nur bis zu einem Maximum möglich. Das proximale Ende 28 der Gewindespindel 29 behält deshalb - auch bei der Einstellung der maximalen Injektionsdosis - einen ausreichenden Sicherheitsabstand vom Kolben 14.

Wie Fig. 14 zeigt, kann auf der Innenseite des Abschnitts 72 (des Gehäuseteils 21) ein Anschlag 76 für das Sperrglied 60 vorgesehen werden, und dieser Anschlag 76 begrenzt die Dosierung auf maximal eine Umdrehung des Stellglieds 23, oder bei Bedarf auch auf einen kleineren Winkelweg, z.B. eine halbe Umdrehung. Hierdurch wird eine Überdosierung mit Sicherheit vermieden.

An dieser Stelle ist darauf hinzuweisen, daß bei rein akustischer Dosierung, also durch Zählen von Klickgeräuschen, die Anordnung vereinfacht werden kann, da dann das Sperrglied 60 und die Längsnut 66 entfallen können; hierbei entfällt dann aber auch die Möglichkeit des Anschlags 76, der eine Überdosierung verhindert. In diesem Fall kann das proximale Ende 28 der Gewindespindel 29 so weit verstellt werden, daß ein ausreichender Sicherheitsabstand zum Kolben 14 nicht mehr gegeben ist. Deshalb wird diese Möglichkeit weniger bevorzugt.

In der Arbeitsstellung steht die Außenverzahnung 55 des Führungsglieds 34 in Eingriff mit der Innenverzahnung 56 des Gehäuseteils 21, wie in den Figuren 3 und 4 dargestellt. In dieser Stellung ist also das Führungsglied 34 drehfest mit dem Gehäuseteil 21 verbunden. Es ist darauf hinzuweisen, daß dies in gleicher Weise auch durch eine Stirnverzahnung am distalen Ende des Führungsglieds 34 möglich wäre, die dann in der Arbeitsstellung in eine entsprechende Stirnverzahnung, oder einfach einen entsprechenden inneren Vorsprung, des Gehäuseteils 21 eingreifen würde. Der Benutzer könnte auch bei einer solchen Ausbildung nur in der Arbeitsstellung dosieren. Auch würde eine rein kraftschlüssige Verbindung zwischen Führungsglied 34 und Gehäuseteil 21 genügen, die nur in der Arbeitsstellung vorhanden sein müßte, z.B. durch entsprechend große Reibung zwischen diesen beiden Teilen, wenn diese sich in der Arbeitsstellung befinden. Es ist klar, daß hier vielfache Variationen möglich sind, die dieselbe Funktion zur Folge haben.

Das Stellglied 23 ist am distalen Ende des Gewindeglieds 38 durch eine Schnappverbindung befestigt. Das Gewindeglied 38 hat hierzu eine radiale Ausnehmung 80 (Fig. 9), in die ein radialer Vorsprung 81 eines federnden axialen Fortsatzes 82 des Stellglieds 23 von innen hineinragt. Das Stellglied 23 hat ferner innen einen ringförmigen axialen Fortsatz 83, der im montierten Zustand das distale Ende des Gewindeglieds 38 formschlüssig umgibt. Bei der Montage braucht also das Stellglied 23 nur auf das distale Ende des Gewindeglieds 28 aufgepreßt zu werden, wobei dann der radiale Vorsprung 81 in die Ausnehmung 80 einschnappt und eine feste Verbindung zwischen diesen Teilen herstellt. Die Befestigung des Gehäuseteils 11 am Gehäuseteil 21 erfolgt in ähnlicher Weise durch eine Schnappverbindung, die beim Zusammenstecken dieser Gehäuseteile 11, 21 automatisch entsteht. Dies ermöglicht eine sehr einfache und preiswerte Montage.

### Arbeitsweise

Das Injektionsgerät gemäß Fig. 1 wird beim Hersteller fertig konfektioniert, d.h. es kommt bereits mit einer gefüllten Kartusche 12 geladen zum Verbraucher. Dieser befestigt in der bereits beschriebenen Weise eine sterile Nadel 15 am Gehäuseteil 11, stellt die beiden Markierungen 71, 72 (Fig. 1) einander gegenüber und zieht dann das Betätigungsglied 23 in distaler Richtung, wie in Fig. 3 dargestellt. Dabei gleitet das Sperrglied 60 durch die Längsnut 66, wie ebenfalls bereits beschrieben.

Erst in der Stellung gemäß Fig. 3 ist nun eine Einstellung der gewünschten Injektionsdosis möglich, und dieses geschieht durch Verdrehen des Stellglieds 23 bis zur gewünschten Dosis (Pfeil 24 in Fig. 4), wobei sich das proximale Ende 28 der Gewindespindel 29 in Richtung des Pfeiles 74 (Fig. 4) verschiebt, also in Richtung zum Kolben 14. Die Dosiseinstellung ist möglich, weil in der Arbeitsstellung gemäß Fig. 3 und 4 das Führungsglied 34 nicht relativ zum Gehäuseteil 21 verdrehbar ist, wie weiter oben ausführlich beschrieben. Fig. 8 zeigt schematisch, wie in dieser Stellung das Führungsglied 34 über seine Außenverzahnung 55 mit der Inennverzahnung 56 des Gehäuseteils 21 in Eingriff steht, so daß es relativ zum Gehäuseteil 21 nicht verdrehbar ist.

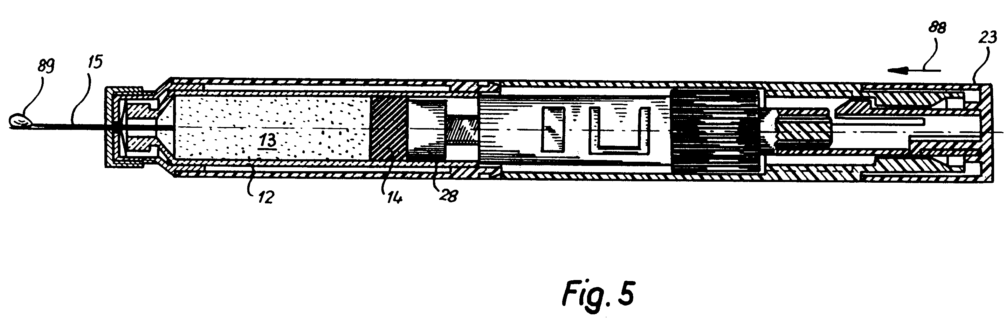

Nach Einstellung der erforderlichen Injektionsdosis sticht der Patient nun die Nadel 15 ein und drückt dann in proximaler Richtung (Pfeil 88 der Fig. 5) auf das Stellglied 23, wodurch der Kolben 14 in proximaler Richtung verschoben wird und Flüssigkeit 13 durch die Nadel 15 in den Patienten injiziert wird. Dies ist in Fig. 5 symbolisch durch den Tropfen 89 angedeutet. Die Injektion ist dann abgeschlossen. Beim Injektionsvorgang wird das Sperrglied 60 durch den kegelstumpfförmigen Abschnitt 70 am distalen Ende des Sperrgürtels 65 federnd radial nach innen ausgelenkt und gleitet unbehindert über den Sperrgürtel 65 hinweg. Dies ist in der umgekehrten Richtung nicht möglich, weil dort die hakenförmige Seite 61 des Sperrglieds 60 gegen eine zu ihr komplementäre steile Schulter 65' am proximalen Ende des Sperrgürtels 65 anliegt, was eine axiale Verschiebung dort verhindert, mit Ausnahme der Drehstellung, in der das Sperrglied 60 durch die Längsnut 66 gleiten kann.

Der Patient stellt nun wieder die beiden Symbole 71, 72 (Fig. 1) einander gegenüber, was durch einen - nicht dargestellten - Anschlag am proximalen Ende der Längsnut 66 erleichtert werden kann, und der ganze Vorgang kann dann wiederholt werden. Dabei ist für jede Injektion eine andere Dosiseinstellung möglich.

In der Stellung nach Fig. 1 hat Herumdrehen am Stellglied 23 keinerlei Einfluß auf die injizierte Dosis, da in dieser Stellung, also der Ruhestellung, sich das Führungsglied 34 relativ zum Gehäuse 21 frei drehen kann und daher eine Drehung des Stellglieds 23 keine Verlängerung des Stößels 29, 38 zur Folge hat. Dieser dreht sich nur mit dem Stellglied 23 mit.

In der Stellung nach Fig. 4 verhindert der Anschlag 76 (Fig. 14), daß das Stellglied 23 um mehr als eine Umdrehung verdreht wird und begrenzt dadurch die einstellbare Dosis nach oben. Der Anschlag 76 kann bei Bedarf auch so gewählt werden, daß er z.B. nur eine Verdrehung des Stellglieds 23 um eine halbe Umdrehung ermöglicht.

Zu den Zeichnungen noch ein besonderer Hinweis: Die raumbildlichen Darstellungen nach den Figuren 6 bis 8 dienen dazu, die Erfindung in einfacher und leicht verständlicher Weise zu erläutern. Sie stimmen mit den Darstellungen nach den Figuren 1 bis 5 und 9 bis 14 nicht in allen Punkten überein. Die Schnittdarstellungen nach den Figuren 2 bis 5 und 9 bis 14 zeigen ein funktionsfähiges Gerät und die bei einem solchen zu beachtenden Maße und Maßverhältnisse.

Wie vorstehend ausführlich an verschiedenen Stellen beschrieben, sind im Rahmen der Erfindung vielfache Abwandlungen und Modifikationen möglich. Zweckmäßig wird das erfindungsgemäße Gerät als Einweggerät ausgebildet, das nach Aufbrauchen der Injektionsflüssigkeit 13 (im Behälter 12) weggeworfen wird. Es besteht aber auch die Möglichkeit, nach dem Verbrauch einer Kartusche 12 eine neue Kartusche einzusetzen und die Gewindespindel 29 wieder ganz in das Gewindeglied 28 zurückzuschrauben. In diesem Fall empfiehlt sich die Form der Ratschenanordnung gemäß Fig. 11, die eine Verdrehung in beiden Richtungen ermöglicht, während die in Fig. 6 dargestellte Form der Ratschenzähne 49 nur eine Verdrehung in einer einzigen Richtung ermöglicht.

## Patentansprüche

1. Injektionsgerät mit einem zur Aufnahme von Injektionsflüssigkeit (13) dienenden Gehäuse (11, 21),
mit einem längenverstellbaren Stößel (29, 38), der beim Injektionsvorgang zwischen einer ersten Stellung, im folgenden Dosierstellung genannt, und einer zweiten Stellung, im folgenden Ruhestellung genannt, verschiebbar ist, um Injektionsflüssigkeit (13) auszupressen (89),
und mit einer Stellanordnung (21, 23),
welche in der Dosierstellung mittels einer dort aktivierten Wirkverbindung (55, 56) eine Veränderung der wirksamen Länge des Stößels (29, 38) und damit eine Dosierung der zu injizierenden Menge von Injektionsflüssigkeit (13) ermöglicht, dadurch gekennzeichnet, daß
in der Ruhestellung diese Wirkverbindung (55, 56), gesteuert durch die Längen-Verschiebung des längenverstellbaren Stößels (29, 38) von der Dosierstellung in die Ruhestellung desaktiviert ist.

2. Injektionsgerät nach Anspruch 1, dadurch gekennzeichnet, daß in der Ruhestellung die Wirkverbindung (55, 56) unterbrochen ist.

3. Injektionsgerät nach Anspruch 1 oder 2, dadurch gekennzeichnet, daß zwischen längenverstellbarem Stößel (29, 38) und Gehäuse (21) eine Sperre (60, 66) vorgesehen ist, welche nur in einer bestimmten Drehstellung oder einem bestimmten Drehstellungsbereich des längenverstellbaren Stößels (29, 38) eine axiale Verschiebung desselben relativ zum Gehäuse (21) von seiner Ruhestellung in seine Dosierstellung ermöglicht.

4. Injektionsgerät nach Anspruch 3, dadurch gekennzeichnet, daß die Sperre (60, 66) bei einer Bewegung des längenverstellbaren Stößels (29, 38) von seiner Dosierstellung in seine Ruhestellung nicht wirksam ist.

5. Injektionsgerät nach Anspruch 3 oder 4, dadurch gekennzeichnet, daß dem Stellglied (23) ein in der Dosierstellung wirksamer Anschlag (76) zugeordnet ist, welcher die einstellbare Dosis nach oben begrenzt.

6. Injektionsgerät nach einem oder mehreren der vorhergehenden Ansprüche, dadurch gekennzeichnet, daß mit dem längenverstellbaren Stößel (29, 38) ein Stellglied (23) zu dessen Verdrehung und/oder axialen Verschiebung verbunden ist.

7. Injektionsgerät nach einem oder mehreren der vorhergehenden Ansprüche, dadurch gekennzeichnet, daß der längenverstellbare Stößel (29, 38) eine in einem Gewinde (35) eines Gewindeglieds (38) geführte Gewindespindel (29) aufweist, mit welchem Gewindeglied (38) ein zur Längsführung der Gewindespindel (29) ausgebildetes Führungsglied (34) drehbar verbunden ist, welches im Gehäuse (21) axial verschiebbar angeordnet ist.

8. Injektionsgerät nach Anspruch 7, dadurch gekennzeichnet, daß zwischen Gewindeglied (38) und Führungsglied (34) eine Ratschenanordnung (48, 49) vorgesehen ist.

9. Injektionsgerät nach Anspruch 7 oder 8, dadurch gekennzeichnet, daß das Führungsglied (34) in der Ruhestellung relativ zum Gehäuse (21) verdrehbar, dagegen in der Dosierstellung relativ zum Gehäuse (21) nicht verdrehbar ist.

10. Injektionsgerät nach Anspruch 9, dadurch gekennzeichnet, daß das Führungsglied (34) in der Dosierstellung form- oder kraftschlüssig (55, 56) mit dem Gehäuse (21) verbunden ist.

11. Injektionsgerät nach Anspruch 9 oder 10, dadurch gekennzeichnet, daß das Führungsglied (34) mit einer Vertiefung oder einem Vorsprung (55) versehen ist, mit der bzw. dem in der Dosierstellung ein hierzu passender Vorsprung bzw. eine hierzu passende Vertiefung (56) des Gehäuses (21) in Eingriff steht, nicht aber in der Ruhestellung, wodurch in der Ruhestellung die Wirkverbindung zur Veränderung der wirksamen Länge des Stößels (29, 38) unterbrochen wird.

12. Injektionsgerät nach einem oder mehreren der Ansprüche 7 bis 11, dadurch gekennzeichnet, daß die Drehung zwischen Gewindeglied (38) und Führungsglied (34) auf eine Drehrichtung beschränkt ist, welche eine Verlängerung der wirksamen Länge des Stößels (29, 38) bewirkt.

13. Injektionsgerät nach den Ansprüchen 8 und 12, dadurch gekennzeichnet, daß die Ratschenanordnung (48, 49) gegen eine Drehung in der unerwünschten Drehrichtung sperrend ausgebildet ist.

## Claims

1. Injection device with a housing (11, 21) serving for holding injection fluid (13), with an adjustable-length tappet (29, 38) which during the injection process is displaceable between a first position, referred to in the following as the dosing position, and a second position, referred to in the following as the rest position, in order to express (89) injection fluid (13), and with an adjusting arrangement (21, 23) which in the dosing position enables alteration of the effective length of the tappet (29, 38) and hence dozing of the quantity of injection fluid (13) to be injected by means of an operative connection (55, 56) activated there, characterised in that in the rest position this operative connection (55, 56) is deactivated controlled by the longitudinal displacement of the adjustable-length tappet (29, 38) from the dosing position to the rest position.

2. Injection device according to claim 1, characterised in that in the rest position the operative connection (55, 56) is interrupted.

3. Injection device according to claim 1 or 2, characterised in that a blocking device (60, 66) is provided between the adjustable-length tappet (29, 38) and the housing (21), which blocking device only allows axial displacement of the adjustable-length tappet relative to the housing (21) from its rest position to its dosing position in a given rotary position or a given rotary position range of the tappet (29, 38).

4. Injection device according to claim 3, characterised in that the blocking device (60, 66) is not effective when the adjustable-length tappet (29, 38) is moved from its dosing position to its rest position.

5. Injection device according to claim 3 or 4, characterised in that a stop (76) effective in the dosing position is associated with the adjusting member (23), said stop providing an upper limit for the dose which can be set.

6. Injection device according to one or more of the preceding claims, characterised in that an adjusting member (23) is connected with the adjustable-length tappet (29, 38) for its rotation and/or axial displacement.

7. Injection device according to one or more of the preceding claims, characterised in that the adjustable-length tappet (29, 38) comprises a threaded spindle (29) guided in a thread (35) of a threaded member (38) with which a guiding member (34) designed for longitudinal guidance of the threaded spindle (29) and disposed displaceable axially in the housing (21) is connected rotatably.

8. Injection device according to claim 7, characterised in that a ratchet arrangement (48, 49) is provided between the threaded member (38) and the guiding member (34).

9. Injection device according to claim 7 or 8, characterised in that the guiding member (34) is rotatable relative to the housing (21) in the rest position, but is not rotatable relative to the housing (21) in the dosing position.

10. Injection device according to claim 9, characterised in that in the dosing position the guiding member (34) is connected positively or frictionally (55, 56) with the housing (21).

11. Injection device according to claim 9 or 10, characterised in that the guiding member (34) is provided with a hollow or a projection (55) with which a matching projection or a matching hollow (56) of the housing (21) engages in the dosing position, but not in the rest position, whereby the operative connection for alteration of the effective length of the tappet (29, 38) is interrupted in the rest position.

12. Injection device according to one or more of claims 7 to 11, characterised in that the rotation between the threaded member (38) and the guiding member (34) is limited to a direction of rotation which causes an increase in the effective length of the tappet (29, 38).

13. Injection device according to claims 8 and 12, characterised in that the ratchet arrangement (48, 49) is embodied so that it blocks rotation in the undesired direction of rotation.

## Revendications

1. Appareil d'injection comportant un boîtier (11, 21) servant à la réception d'un liquide d'injection (13), présentant un poinçon (29, 38) réglable quant à sa longueur qui est déplaçable lors de l'opération d'injection entre une première position, appelée dans ce qui suit position de dosage, et une seconde position, appelée dans ce qui suit position de repos, pour chasser (89) du liquide d'injection (13), et comportant un organe de réglage (21, 23) qui permet dans la position de dosage, au moyen d'une liaison d'action (55, 56) qui y est activée, de modifier la longueur active du poinçon (29, 38) et ainsi un dosage de la quantité à injecter du liquide d'injection (13), caractérisé en ce que dans la position de repos, cette liaison d'action (55, 56) est désactivée sous la commande du déplacement longitudinal du poinçon (29, 38) réglable quant à sa longueur depuis la position de dosage à la position de repos.

2. Appareil d'injection selon la revendication 1, caractérisé en ce que la liaison d'action (55, 56) est interrompue dans la position de repos.

3. Appareil d'injection selon l'une ou l'autre des revendications 1 ou 2, caractérisé en qu'il est prévu entre le poinçon (29, 38) réglable quant à sa longueur et le boîtier (21) un dispositif de blocage (60, 66) qui permet un déplacement axial du poinçon (29, 38) réglable quant à sa longueur par rapport au boîtier (21) uniquement dans une position en rotation déterminée ou dans une plage déterminée de positions en rotation du poinçon, pour venir depuis la position de repos à la position de dosage.

4. Appareil d'injection selon la revendication 3, caractérisé en ce que le dispositif de blocage (60, 66) n'est pas actif lors d'un mouvement du poinçon (29, 38) réglable quant à sa longueur depuis sa position de dosage à sa position de repos.

5. Appareil d'injection selon l'une ou l'autre des revendications 3 et 4 caractérisé en ce qu'à l'organe de réglage (23) est associée une butée (76) active dans la position de dosage, qui limite la dose réglable vers le haut.

6. Appareil d'injection selon l'une ou plusieurs des revendications précédentes, caractérisé en ce qu'un organe de réglage (23) est relié au poinçon (29, 38) réglable quant à sa longueur, pour sa rotation et/ou son déplacement axial.

7. Appareil d'injection selon l'une ou plusieurs des revendications précédentes, caractérisé en ce que le poinçon (29, 38) réglable quant à sa longueur présente une broche filetée (29) menée dans le pas de vis (35) d'un organe taraudé (38), auquel est relié en rotation un organe de guidage (34) réalisé pour le guidage longitudinal de la broche filetée (29) et agencé de façon axialement mobile dans le boîtier (21).

8. Appareil d'injection selon la revendication 7, caractérisé en ce qu'il est prévu entre l'organe taraudé (38) et l'organe de guidage (34) un agencement à cliquet (48, 49).

9. Appareil d'injection selon l'une ou l'autre des revendications 7 et 8, caractérisé en que l'organe de guidage (34) peut être tourné par rapport au boîtier (21) dans la position de repos, mais qu'il ne peut pas être tourné par rapport au boîtier (21) dans la position de dosage.

10. Appareil d'injection selon la revendication 9, caractérisé en ce que dans la position de dosage, l'organe de guidage (34) est relié au boîtier (21) en coopération de formes ou de forces (55, 56).

11. Appareil d'injection selon l'une ou l'autre des revendications 9 et 10, caractérisé en ce que l'organe de guidage (34) est pourvu d'une cavité ou d'une saillie (55) avec laquelle, dans la position de dosage, est en engagement une saillie ou cavité (56) du boîtier (21) adaptée à celle-ci, mais non pas dans la position de repos, suite à quoi la liaison d'action pour modifier la longueur effective du poinçon (29, 38) est interrompue dans la position de repos.

12. Appareil d'injection selon l'une ou plusieurs des revendications 7 à 11, caractérisé en ce que la rotation entre l'organe taraudé (38) et l'organe de guidage (34) est limitée à une seule direction de rotation qui provoque un allongement de la longueur effective du poinçon (29, 38).

13. Appareil d'injection selon l'une ou l'autre des revendications 8 et 12, caractérisé en ce que l'agencement à cliquet (48, 49) est réalisé de manière à bloquer une rotation dans la direction de rotation non désirée.
